# EUROPEAN PATENT SPECIFICATION

(11) **EP 2 344 141 B1**
(45) Date of publication and mention of the grant of the patent: **01.05.2019**
(21) Application number: 09748467.9
(22) Date of filing: 30.09.2009
(51) Int. Cl.: A61K 9/20, A61K 31/138, A61K 31/4422

(54) **COMPOSITIONS COMRRISING AMLODIPINE AND BISOPROLOL**
ZUSAMMENSETZUNGEN MIT AMLODIPIN UND BISOPROLOL
COMPOSITION PHARMACEUTIQUE CONTENANT DE L'AMILODIPINE ET DU BISOPROLOL

(30) Priority: 30.09.2008 HU 0800591
(43) Date of publication of application: 20.07.2011
(60) Divisional application: 11003486.5
(73) Proprietor: Egis Gyógyszergyár Zrt., 1106 Budapest (HU)
(72) Inventor: WAGNER, László, H-1172 Budapest (HU); ZSIGMOND, Zsolt, H-2234 Maglód (HU); UJFALUSSY, György, H-1028 Budapest (HU); LEVENTISZNÉ HUSZÁR, Magdolna, H-1125 Budapest (HU); TONKA-NAGY, Péter, H-1171 Budapest (HU); BÁRCZAY, Erzsébet, H-1165 Budapest (HU); GÓRA, Lászlóné, H-2117 Isaszeg (HU); SZELECZKI, Edit, H-1165 Budapest (HU); FÜLÖP, Ágnes, H-1174 Budapest (HU)
(74) Representative: Stolmár & Partner Patentanwälte PartG mbB
(86) International application number: PCT/HU2009/000085
(87) International publication number: WO 2010/038091

(56) References cited:
- WO-A1-2005/099699
- DE-U1- 20 116 428
- IN-A1- 224 215
- BURGES R A ET AL: "PHARMACOLOGIC PROFILE OF AMLODIPINE" AMERICAN JOURNAL OF CARDIOLOGY, CAHNERS PUBLISHING CO., NEWTON, MA, US LNKD- DOI:10.1016/0002-9149(89)90956-9, vol. 64, no. 17, 7 November 1989 (1989-11-07), pages 10I-20I, XP000946560 ISSN: 0002-9149
- ABDOH A ET AL: "AMLODIPINE BESYLATE-EXCIPIENTS INTERACTION IN SOLID DOSAGE FORM", PHARMACEUTICAL DEVELOPMENT AND TECHNOLOGY, NEW YORK, NY, US, vol. 9, no. 1, 1 January 2004 (2004-01-01) , pages 15-24, XP008034356, ISSN: 1083-7450, DOI: 10.1081/PDT-120027414

## Description

### FIELD OF THE INVENTION

The present invention relates to a stable solid pharmaceutical composition containing a homogenized powder mixture comprising amlodipine of the formula 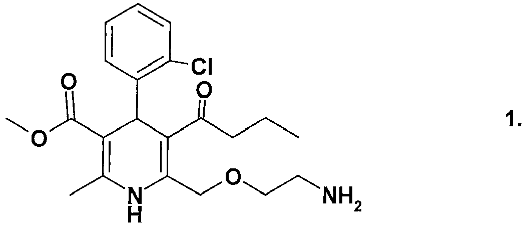 and bisoprolol as active ingredients.
More particularly, the present invention relates to a composition packed in a damp-proof package containing a homogenized powder mixture of, preferably, amlodipine besylate and bisoprolol fumarate of the formula 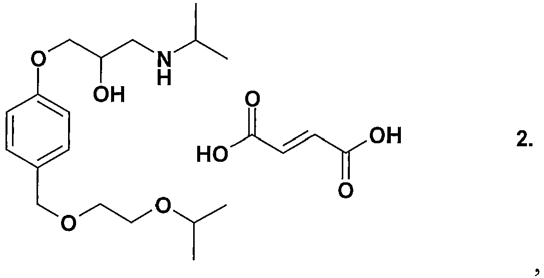 further a filler, a disintegrant, a lubricant and an anti-adhesion agent used in the pharmaceutical industry, furthermore, in which
the amount of the compound of *N*-(2-{[4-(2-chlorphenyl)-3-(ethoxycarbonyl)-5-(methoxycarbonyl)-6-methyl-1,4-dihydro-2-pyridinyl)-methoxy}-ethyl)-asparaginic acid of the formula 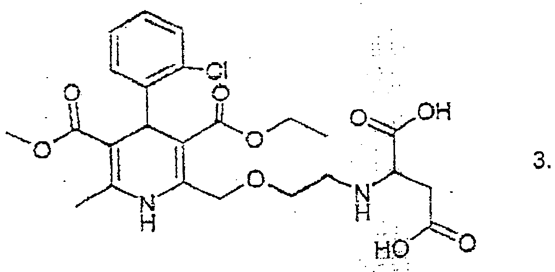 does not exceed 0.5% during the preparation and storage of the composition.

### BACKGROUND OF THE INVENTION

The required blood pressure value for reducing the risk of cardiovascular morbidity and mortality can be achieved by a medicine consisting of a single compound or the optimal combination of two compounds according to the practice of "evidence based" therapy.

A combination therapy is more effective, generally lower amounts of each compound are needed than in case of a monotherapy, therefore the side effects decrease and the compliance of patients increases.

Due to these advantages the role of the combination therapy of pharmaceuticals grows in the treatment of hypertension and complications thereof. Combinations of beta blockers, diuretcs, ACE inhibitors, ARB-s, respectively calcium channel blockers are used typically.

Recently, for better "patient compliance" and reduction of costs of therapy these combinations are marketed more and more frequently as so called "fix combination" forms comprising both active ingredients in a single dosage form.

The use of fix combinations is recommended unequivocally by international guidelines.

Several fix combinations comprising calcium channel blockers ("calcium antagonists") and beta blockers used preferably for the treatment of hypertension and angina pectoris are recently on the market, such as tablets containing felodipine and metoprolol or nifedipine and atenolol.

The fix combination of amlodipine and bisoprolol has not been marketed yet, but several articles and patent applications deal with the combination thereof. The international patent application No. WO2005/099699 discloses the combinations of S-amlodipine and beta blockers including the combination with bisoprolol.

The application mentions several pharmaceutical solutions for the co-formulation of these ingredients, but is silent about the main problem of the practical applicability, namely the chemical incompatibility of both active ingredients.

In the so called "mono-compositions" containing only one active ingredient, amlodipine is in the form of besylate salt, and bisoprolol is used as fumarate salt.

The formulation of amlodipine besylate and bisoprolol fumarate into one single dosage form seems to be practical, because both bisoprolol fumate and amlodipine besylate are suitable for the preparation of stable pharmaceutical dosage forms.

According to the Indian patent application No.845/MUM/2004 amlodipine besylate and bisoprolol fumarate interact. Due to our experiments the product is the compound of *N*-(2-{[4-(2-chlorphenyl)-3-(ethoxycarbonyl)-5-(methoxycarbonyl)-6-methyl-1,4-dihydro-2-pyridinyl]-methoxy}-ethyl)-asparaginic acid of the formula (3). This compound is formed by the chemical reaction of amlodipine base and fumaric acid. Formation of this product is unexpected because US patent application No. 6518288 states that the salt of amlodipine formed with fumaric acid is stable and does not transform into the compound of formula (3).

The current requirements of international pharmaceutical authorities accept very low - some tenths of a weight percent - limits of the degradation products of pharmaceutical compositions only.

The absorption properties and pharmacokinetic effects of the above-mentioned salts are well known. The patients are accustomed to the use of compositions containing these salts and they have become habituated to the effects thereof.

There is a need for a stable solid dosage form which comprises amlodipine or a pharmaceutically acceptable salt thereof, preferably amlodipine besylate and bisoprolol fumarate. The composition should be a tablet or a capsule in which the amount of the contaminant arising out of the incompatibility of both active ingredients is kept at a low level during storage too.

According to the Indian patent application No. 845/MUM/2004 a combination composition of amlodipine besylate and bisoprolol fumarate can be prepared only if the active ingredients are separated into different granules. The granules are mixed with further excipients and filled in capsules or sachettes, or pressed into so called bilayered tablets. The essence of the solution of the Indian invention is the separation of both ingredients from each-other. With this, the physical contact between the active ingredients is essentially prevented, which could help the formation of the reaction product of the formula (3) during the blending of granules and especially during the tablet pressing process with the intensive contact of large surfaces.

The process according to the Indian patent application has several disadvantages. The individual granulation and homogenisation of the active ingredients increase the number of required technological steps. The preparation of bilayer tablets requires difficult and special apparatus.

There is a need for a stable solid dosage form which comprises amlodipine or a pharmaceutically acceptable salt thereof, preferably amlodipine besylate and bisoprolol fumarate. The composition should be a tablet or a capsule in which the amount of the contaminant arising out of the incompatibility of both active ingredients is kept at a low level even during the storage and does not require separated treatment of the active ingredients either during the production process or in the composition.

### SUMMARY OF THE INVENTION

We found surprisingly that in case of an appropriate selection of the packaging and the circumstances a stable pharmaceutical composition can be prepared, which, if packed satisfactorily, meets the requirements of the severe safety regulations concerning the pharmaceuticals and the amount of *N*-(2-{[4-(2-chlorphenyl)-3-(ethoxycarbonyl)-5-(methoxycarbonyl)-6-methyl-1,4-dihydro-2-pyridinyl]-methoxy}-ethyl)-asparaginic acid of the formula (3) does not exceed 0.5% in the composition either during the preparation, or during storage until the expiry date of the pharmaceutical composition, at least two years.

The present invention relates to a stable solid pharmaceutical composition containing a homogenized powder mixture of amlodipine base or a pharmaceutically acceptable salt thereof and bisoprolol fumarate and pharmaceutically accepted excipients packaged in a damp-proof package and further comprising less than 0.5 % of the compound of the formula (3) based on the weight of the active ingredients, wherein the composition further comprises an organic or inorganic filler, disintegrant, lubricant and optionally a binding agent.

### DETAILED DESCRIPTION OF THE INVENTION

During our experiments we found that both active ingredients interact even in a simple powder mixture and the reaction product is formed in a considerable amount. The incompatibility
is more explicit if both ingredients are placed in a low-porosity dosage form, e.g. in a tablet in which their crystals contact each other under high pressure on a large area.

The interaction strongly depends on the temperature: the amlodipine and bisoprolol content in a mixture of amlodipine besylate and bisoprolol fumarate varies after one month storage at different temperatures as follows:

**Table 1**

| | 25°C/ 1 month | 40°C/ 1 month | 70°C/ 1 month |
|---|---|---|---|
| Amlodipine | ∼100% | 59% | 25% |
| Bisoprolol | ∼100% | 85% | 71% |

For the person skilled in the field of the pharmaceutical technology it is obvious that in case of forming a composition containing two incompatible ingredients, these ingredients have to be separated with a suitable process. For this purpose the coating of one active ingredient with a polymer excipient can be enough. The polymer layer of the coating can form suitable isolation layer between the crystal surfaces of both active ingredients.

Our formulation experiments have failed. The active ingredients are heat sensitive. On one hand, the heat sensitivity of the active ingredients causes problems for not only the aqueous coating processes, but for the coating processes using organic solvents as well, because the solvent elimination entails thermal stress. On the other hand, during the drying process traces of humidity remain in the composition, which also helps the interaction of the active ingredients.

We found surprisingly that preventing the incompatibility between both active ingredients and self heat-degradations of the active ingredients is not enough to exclude processes using water or organic solvents accompanied by heat stress.

Preventing adverse effects of heat stress, combination tablets were prepared by a direct pressing process. If tablets were stored in a glass vessel closed by a polyethylene cap at 30 °C in 65 % relative humidity for three months, the degradation products remained under the detection limit.

In case the same tablets were stored in a PVC/PVdC-type (polyvinylchlorid/polyvinylydenchloride type) thermoformable blister foil closed with an aluminium foil at 30 °C in 65 % relative humidity for three months, we found surprisingly that the minimal humidity which permeated through the PVC/PVdC-type blister foil - which is known as more damp-proof than PVC - was enough to the formation of an unacceptable high amount of contamination.

It turned out that it is not enough to protect the product from the humidity during the production process, but during the storage the environment has to be kept dry as well.

These experiments indicate that for starting the interaction between the ingredients in a solid phase, the presence of extremely small amount of water absorbed on surfaces of crystals is enough. In the composition according to the present invention the reactions, which cause the formation of the contaminant, do not take place at all or only in a very limited range.

According to the present invention there is provided a stable solid packed pharmaceutical composition of amlodipine and bisoprolol as active pharmaceutical ingredients, which comprises less than 0.5%, preferably less than 0.3%, more preferably less than 0.2% of the compound of the formula (3), is packed in a damp-proof package containing a homogenized powder mixture of amlodipine base or a pharmaceutical acceptable salt thereof, preferably amlodipine besylate, bisoprolol fumarate, further a
pharmaceutically accepted filler, disintegrant, lubricant, anti-adhesive agents, optionally binding agents.

According to our recognition amlodipine reacts with fumaric acid. During the reaction resulting in the formation of the contaminant the other salt forming component (e.g. anion of the benzolsulfonic acid) does not play any role. Therefore according to the present invention amlodipine can be used in a base or in a salt form (e.g. amlodipine besylate).

The composition according to the present invention is of fundamental importance from the point of view of the stability of the pharmaceutical composition.

For the formulation of the tablets by dry process such excipients have to be used which have appropriate pressable and free-flowing properties beyond their main function.

According to the present invention all compounds of the composition except the active ingredients are excipients.

Terms referring to an excipient such as filler, disintegrant, lubricant, anti-adhesive and binding agents relate to the categories of the excipients. Therefore they also relate to the mixtures of the appropriate excipients and composites thereof, moreover to the common use of excipients from the same category in the same composition. Such case is, e.g. if as filler two different fillers applicable in the pharmaceutical industry are used in the composition, such as lactose and microcrystalline cellulose as composite.

The terms referring to the excipients above also relate to cases, in which a composition comprises several excipients from different categories. These cases are for example if a filler is used beside the disintegrant, e.g. lactose as a composite with povidon and copovidon, or if a glidant or anti-adhesive agent is used commonly with the filler, e.g. as a composite of microcrystalline cellulose with colloidal silicon dioxide.

According to the present invention the use of such composites is equivalent with the common use of different components, therefore these technical solutions also form a part of the present invention.

As filler agents the composition according to the present invention may contain fillers used in the pharmaceutical industry or mixtures thereof, preferably microcrystalline cellulose, anhydrous calcium hydrogen phosphate, spray dried lactose, or mannitol or a mixture thereof, most preferably low moisture grades of microcrystalline cellulose.

In the course of our tabletting experiments we found that the lowest rate of degradation has been detected in case of use of microcrystalline cellulose (low moisture grades of microcrystalline cellulose were used preferably; see Table 2.)

**Table 2**

| Effects of fillers for the degradation and the rate of interaction after one month storage at 50 °C (mixtures of amlodipine besylate/bisoprolol fumarate/filler agent in a rate: 0.1:0.1:5) are as follows: | | | | |
|---|---|---|---|---|
| | Fillers | | | |
| | Microcrystalline cellulose (Vivapur) | Anhydrous potassium hydrogen phosphate (Emcompress) | Lactose DC 11 (Tablettose) | DC Mannitol (Perlitol) |
| Compound of the formula (3) | 0.06 % | 0.46 % | 0.24 % | 0.09 % |
| All contaminants | 0.29 % | 0.77 % | 0.56 % | 0.31 % |

Rates of the filler agents suitable for a direct compressing process in the tablets according to the present invention are 60%-90%, preferably 70%-90%, more preferably 80-90%.

The use of a filler in case of capsule products is not necessary. In order to ensure that the active ingredients be spread evenly in one production batch and thereby always the required amount of both active ingredients be filled in each capsule under the whole encapsulation process it is preferable to use an appropriate amount of a filler having a high specific surface area which prevents the segregation of ingredients during the process.

Such fillers having a high specific surface area are e.g. powdered cellulose or microcrystalline cellulose. Microcrystalline cellulose is preferable even from the point of view that the filled composition in the capsule is kept together by a relative low thickener pressure due to its mechanical interlocking bindings and accordingly prevents the dispersion of the substances of the composition during the encapsulation process.

For these above-mentioned reasons the optimal amount of the filler in an encapsulated product is 10-75%, preferably 45%-75%, more preferably 55%-65% based on the weight of the filled substance of the composition.

The use of low moisture grades of fillers is more preferable according to the present invention.

In addition to their main functional characteristics the excipients used for the preparation of the tabletted compositions by a dry process should have also acceptable pressable and free-flowing properties.

A disintegrant has to be used in case of both tabletted and encapsulated compositions which ensures the quick disintegration of the tablet or capsule due to effects of the digestive juices, thereby the dissolution and absorption of the active ingredients take place quickly and entirely.
Any disintegrant used generally in the pharmaceutical industry can be used as disintegrant. A large selection of disintegrants especially suitable for a direct pressure tabletting process is available for the person skilled in the art.

Thus, the composition of the present invention can comprise as disintegrant any of the disintegrants generally used in the pharmaceutical industry or a mixture thereof, preferably crosspovidon, sodium starch glycolate, croscaramellose, low substituted hydrxypropylcellulose or a mixture thereof, most preferably sodium starch glycolate. According to the present invention the optimal amount of disintegrants in the compositions are 1%-10%, preferably 4%-6% based on the weight of the tablet or the filling substance of the capsule.

The use of anti-adhesives and glidants is necessary both in the tabletted and encapsulated compositions in which the anti-adhesives prevent the wetting of filling agents susceptible to be hygroscopic and the adhesion of the particles thereof to each other by their intense hygroscopic properties.

Thus, as anti-adhesive the composition of the present invention can comprise any types of anti-adhesives generally used in the pharmaceutical industry or a mixture thereof. Preferably it comprises siloids or colloidal silicon dioxides or the mixture thereof. Most preferably it comprises colloidal silicon dioxide. The optimal amount of anti-adhesives in the compositions according to the present invention is 0.3%-2%, preferably 0.5%-1%.

The use of so-called lubricants which reduce the sticking properties and friction is necessary in case of the preparation of both tabletted and encapsulated compositions for facilitating the extrusion of the tablets or the filling substance of the capsules from the mould (tool) and preventing the sticking of the components to the forming or filling tools.

As lubricant, the compositions according to the present invention can comprise any types of lubricants used in the pharmaceutical industry or a mixture thereof. Preferably, they comprise magnesium stearate, sodium stearyl fumarate, glyceryl behenate or a mixture thereof. Most preferably they comprise magnesium stearate. According to the present invention the optimal amount of lubricants in the compositions is 0.5%-3%, preferably 1%-2%.

The solid dosage form according to the present invention is preferably a tablet or a capsule.

Unexpectedly, in a preferable case no binding agents used in the pharmaceutical industry are required for the formulation of the tablets. If it becomes necessary due to a change in the properties of the tablet, e.g. the hardness thereof has increased, any binding agent, e.g. polyvinylpyrrolydone, starch etc. can be used as binding agent.

In case the solid dosage form of the present invention is a tablet, according to the preferable embodiment of the present invention the tablets packaged into a damp-proof protecting package comprise 2%-20%, preferably 2%-10%, most preferably 1%-6% of amlodipine base or a pharmaceutical salt thereof, preferably amlodipine besylate, 2%-20%, preferably 2%-10%, most preferably 1%-6% of bisoprolol fumarate, further 60%-90%, preferably 70%-90%, more preferably 80%-90% of filler, 1%-10%, preferably 4%-6% of disintegrant, 0.5%-3%, preferably 1%-2% of lubricant, 0.3%-2%, preferably 0.5%-1% of anti-adhesive agent and if necessary 1%-10%, preferably 0.1%-5% of binding agent, calculated on the basis of the weight of the tablets.

In case the solid dosage form of the present invention is a capsule, according to the preferable embodiment of the present invention the capsules packaged into a damp-proof protecting package comprise 5%-80%, preferably 5%-18%, most preferably 10%-15% of amlodipine base or a pharmaceutical salt thereof, preferably amlodipine besylate, 5%-80%, preferably 5%-15%, most preferably 1%-10% of bisoprololfumarate, further 1%-10%, preferably 4%-6% of disintegrant, 0.5%-3%, preferably 1%-2% of lubricant, 0.3%-2%, preferably 0.5%-1% of anti-adhesive agent and if necessary 10%-75%, preferably 45%-75%, more preferably 55%-65% of filler, calculated on the basis of the weight of the filling substance of the capsules.

In the packaged dosage forms according to the present invention the pharmaceutical composition containing the active ingredients, e.g. tablets or capsules, are in a package which isolates the dosage forms from the environment, thus protecting the dosage forms from the effects of environment until being administered.

As examples different packaging materials are mentioned with the following abbreviations:
CFF- cold form foil
OPA/AL/PVC foil (oriented polyamid/aluminium/polyvinylchlorid foil).
PVC/PE/PVdC fólia (polyvinylchlorid /polyethylene/ polyvinylydenechlorid foil)
PVC/PCTFE foil (polyvinylchlorid/polychlorotrifluoroethylene foil)
PVdC (polyvinylydenechlorid) layer/foil
PVC (polyvinylchlorid) foil

The damp-proof package of the solid dosage form of the present invention is a hermetically closed vessel or a damp-proof so-called cold-blister (CFF) or thermoformed blister.

According to one embodiment of the present invention the damp-proof packaging unit can be a vessel having a closing component (e.g. vial, plastic box, a glass vessel having an airtight polyethylene or polypropylene cap, polypropylene gallipot etc.), in which one or more tablets or capsules are placed.

Vessels containing tablets or capsules can contain beside capsules and tablets such auxiliary agents which are capable to bind the humidity of the inside atmosphere of the vessel if necessary, such compounds for the binding of humidity are e.g. zeolites or silica gel. Auxiliary agents for binding the humidity can be placed directly among the capsules or tablets, but they can be placed in a separated vessel or in a bag, which are permeable by air and placed among the dosage forms. Such vessels are also suitable for packaging of the solid dosage forms according to the present invention, in which the dosage form and the humidity binding compound are in two separated parts, and the air change is ensured.
In the atmosphere of the package there can be air, inert gases or, if it is suitable, vacuum.

According to an embodiment of the present invention the damp-proof protecting package is a so-called cold blister.
The cold blister is a packaging form, in which the blister is coldly formed from a composite foil and is covered with an aluminium lidding foil. Such composite foil can be the OPA/AL/PVC (oriented polyamid/aluminium/polyvinylchlorid foil). According to an embodiment of the present invention the dosage forms containing the active ingredients, e.g. tablets or capsules, are packaged into a so called cold-blister.

According to another embodiment of the present invention the damp-proof package is e.g. a blister prepared from a thermoformable, damp-proof composite foil, which is covered with an aluminium lidding foil.

According to a further embodiment of the present invention the dosage form containing the active ingredients, e.g. tablets or capsules, is packaged into a blister prepared from a thermoformable, damp-proof composite, which is covered with an aluminium lidding foil. Such a damp-proof composite foil can be e.g. PVC/PE/PVdC, a so-called "triplex foil" or PVC/PCTFE foil.

According to the most preferable embodiment of the present invention the dosage form is a tablet packaged in a cold-formed blister of OPA/AL/PVC composite foil and covered with an aluminium lidding foil (so called cold blister /CFF/), or in a blister of thermoformable, damp-proof composite foil (e.g. PVC/PE/PVdC triplex foil, or PVC/PCTFE foil) covered with an aluminium lidding foil, or in a glass or polypropylene vessel having an airtight container cap of polyethylene or polypropylene, which tablet comprises 1%-6% of amlodipine besylate, 1%-6% of bisoprolol fumarate, 80%-90% of microcrystalline cellulose, 4%-6% of sodium starch glycolate, 1%-2% of magnesium stearate, 0.5%-1% of colloid silica based on the total weight of the tablet.

According to another very preferable embodiment of the present invention there is provided a capsule packaged in a cold-formed blister of OPA/AL/PVC composite foil and covered with an aluminium lidding foil (so-called cold blister /CFF/), or in a blister of thermoformable damp-proof composite foil (e.g. PVC/PE/PVdC triplex foil, or PVC/PCTFE foil) covered with an aluminium lidding foil, or in a glass or polypropylene vessel having an airtight cap of polyethylene or polypropylene, which capsule comprises 10%-15% of amlodipine besylate, 10%-15% of bisoprolol fumarate, 55%-65% of microcrystalline cellulose, 4%-6% of sodium starch glycolate, 1%-2% of magnesium stearate, 0.5%-1% of colloid silica based on the total weight of the filling substance of the capsule.

The composition according to the present invention contains less than 0.5%, preferably an amount between 0.01% and 0.5% of *N-*(2-{[4-(2-clorphenyl)-3-(ethoxycarbonyl)-5-(methoxycarbonyl)-6-methyl-1,4-dihydro-2-pyridinyl]-methoxy}-ethyl)-asparaginic acid. According to a preferable embodiment of the present invention the amount of the asparagin acid derivative compound is less than 0.3%, more preferably an amount between 0.01%-0.3%. According to a more preferable embodiment of the present invention the amount of the asparagin acid derivative is less than 0.2%, more preferably between 0.01%-0.2%. A person skilled in the art can prepare a product containing the compound of the formula (3) in an undetectable amount using the essential features of the present invention. Therefore the scope of protection of the present invention comprises the products, in which the essential features of the present inventions are used and the product contains an undetectable amount of the compound of the formula (3).

In the course of the preparation of the compositions according to the present invention the amlodipine base or a pharmaceutically acceptable salt thereof, preferably amlodipine besylate, bisoprolol fumarate, further organic or inorganic filler, disintegrant, lubricant and optionally binding agent used in pharmaceutical industry are homogenized in a known manner, then an adhesive is added and homogenized
A.) the obtained homogenizate is pressed into tablets in a known manner, or
B.) filled into hard gelatine capsules in a known manner, then the obtained tablets or capsules are packaged into a damp-proof package in a known manner.

If necessary, homogenisation may be preceded by screening to achieve the uniform grain-size of the components.
For the screening a 250 µm sieve can be used preferably. Homogenisation can be carried out in any equipment suitable for homogenisation, preferably in a drum mixer. Tablets can be pressed using any kind of equipment for the preparation of direct compressing tablets in a known manner. Capsules can be prepared in a known manner using any suitable equipment for the encapsulation process.

Tablets or capsules are packaged into suitable blisters or vessels in a known manner.

Tablets are prepared by using 2%-20%, preferably 2%-10%, most preferably 1%-6% of amlodipine base or a pharmaceutically accepted salt thereof, preferably amlodipine besylate, 2%-20%, preferably 2%-10%, most preferably 1%-6% of bisoprolol fumarate, further 60%-90%, preferably 70%-90%, most preferably 80%-90% of filler, 1%-10%, preferably 4%-6% of disintegrant, 0.5-3%, preferably 1%-2% of lubricant, 0.3-2%, preferably 0.5%-1% of anti-adhesive, and if necessary 1%-10%, preferably 0.1-5% binding agent.

Capsules are prepared by using 5%-80%, preferably 5%-18%, most preferably 10%-15% of amlodipine base or a pharmaceutically accepted salt thereof, preferably amlodipine besylate, 5%-80%, preferably 5%-15%, most preferably 10%-15% of bisoprolol fumarate,
further 1%-10%, preferably 4%-6% of disintegrant, 0.5-3%, preferably 1%-2% of lubricant, 0.3-2%, preferably 0.5%-1% of anti-adhesive, and if necessary 10%-90%, preferably 45%-75%, most preferably 55%-65% of filler based on the weight of the filling substance of the capsules.

Microcrystalline cellulose, anhydrous calcium hydrogen phosphate, spray dried lactose or mannitol, preferably microcrystalline cellulose or a mixture thereof, more preferably microcrystalline cellulose having a low humidity can be used as filler, crosspovidon, sodium starch glycolate, croscaramellose, low substituted hydroxypropylcellulose or a mixture thereof, preferably sodium starch glycolate can be used as disintegrant, magnesium stearate, sodium stearyl fumarate, glyceryl behanate or a mixture thereof, preferably magnesium stearate can be used as lubricant for the preparation of tablets or capsules according to the present invention. Siloids or colloid silica or a mixture thereof, preferably colloid silica are used as anti-adhesive agents and if necessary polyvinylpyrrolidone or starch is used as binding agent.

Tablets or capsules are packaged preferably into a cold-formed blister of OPA/AL/PVC composite foil of 130 µm thickness covered with an aluminium lidding foil of 20 µm thickness (so-called cold blister /CFF/), or in a blister prepared from thermoformable, damp-proof composite foil covered with an aluminium lidding foil of 20 µm thickness, or into vessels equipped with an airtight container cap of polyethylene or polypropylene. Most preferably the composition is packed into a so-called cold blister (CFF).

Another embodiment of damp-proof packaging is, as mentioned above, to pack the tablets or capsules together with drying compounds, which bind the humidity. These packaging forms and auxiliary agents are described above in particulars.

According to the most preferable process for the preparation of a solid pharmaceutical dosage form of tablets 1%-6% of amlodipine besylate, 1%-6% of bisoprololfumarate, 80%-90% of microcrystalline cellulose, 4%-6% of sodium starch glycolate, 1%-2% of magnesium stearate are homogenised, then 0.5%-1% of colloid silica is added and homogenized, then the homogenizate is pressed into tablets using a direct compressing process in a known manner, the obtained tablets are packaged into a cold-formed blister of OPA/AL/PVC composite foil and covered with aluminium lidding foil (so-called cold blister /CFF/), or into a blister of thermoformable, damp-proof composite foil, e.g. PVC/PE/PVdC triplex foil, or PVC/PCTFE foil, covered with an aluminium lidding foil, or packaged into a glass or polypropylene vessel having an airtight cap of polyethylene or polypropylene, preferably the tablets are packaged into a cold-formed blister of OPA/AL/PVC composite foil covered with an aluminium lidding foil (so-called cold blister /CFF/).

According to the most preferable process for the preparation of a solid pharmaceutical dosage form of capsules 10%-15% of amlodipine besylate, 10%-15% of bisoprolol fumarate, 55%-65% of microcrystalline cellulose, 4%-6% of sodium starch glycolate, 1%-2% of magnesium stearate based on the total weight of the filling substance are homogenised, then 0.5%-1% of colloid silica is added and homogenized, then the homogenizate is encapsulated into hard gelatine capsules in a known manner and packaged into a cold-formed blister of OPA/AL/PVC composite foil and covered with an aluminium lidding foil (so-called cold blister /CFF/), or into a blister of thermoformable, damp-proof composite foil, e.g. PVC/PE/PVdC triplex foil, or PVC/PCTFE foil, covered with an aluminium lidding foil, or into a glass or polypropylene vessel having an airtight container cap of polyethylene or polypropylene, preferably the tablets are packaged into a cold-formed blister of OPA/AL/PVC composite foil covered with an aluminium lidding foil (so-called cold blister /CFF/).
The advantage of the present invention is that it makes the use of the simple and cheap direct tabletting process possible, even in case of the composition according to the present invention containing incompatible active ingredients. The content of the compound (3) does not exceed 0.5% for at least 2 years in the composition according the present invention.
In case of the use of combination composition the "patient compliance" is better, therefore these compositions are more advantageous than compositions used as monotherapy.
The present invention is demonstrated in the examples below

### Example 1

**Composition for 1000 tablets:**

| | |
|---|---|
| Amlodipine besylate | 13.9 g |
| Bisoprolol fumarate | 10.0 g |
| Microcrystalline cellulose | 265.1 g |
| Sodium starch glycolate | 10.0 g |
| Magnesium stearate | 4.0 g |
| Colloidal silica | 2.0 g |

### Process:

Both active ingredients are sieved using a screen of 250 µm mesh, then homogenized with microcrystalline cellulose, sodium starch glycolate and colloid silica in a drum mixer for ten minutes. Then the magnesium stearate is added to the mixture and the obtained mixture is homogenised for further two minutes.
The homogenizate is pressed into tablets having 305 mg weight with a tablet pressing machine.
The tablets are packaged into a cold-formed blister of OPA/AL/PVC composite foil of 130 µm thickness and covered with an aluminium lidding foil of 20 µm thickness.

The contaminant examination results of the tablets prepared according to the Example 1 at the manufacturing date and after 3 months storage at 40 °C/75% relative humidity are as follows:

| Contaminant | At the production date | After 3 months (40 °C/75 % relative humidity) | After 6 months (40 °C/75 % relative humidity) |
|---|---|---|---|
| *N*-(2-{[4-(2-clorphenyl)-3-(ethoxycarbonyl)-5-(methoxycarbonyl)-6-methyl-1,4-dihydro-2-pyridinyl]-methoxy}-ethyl)-asparaginic acid | < 0.02 % | 0.15 % | 0.26% |

Further quality data of the product of Example 1 are as follows:

| | At the production date | After 3 months (40 °C/75 % relative humidity) |
|---|---|---|
| Disintegration time: | 1' | 1' |
| Dissolution of the active ingredient (15 minute) | | |
| Amlodipine besylate: | 103.1 % | 97.9 % |
| Bisoprolol fumarate: | 102.6 % | 98.9 % |
| Fracture strength | 104 N | 113 N |
| Friction loss: | 0 % | 0 % |
| Humidity (Karl-Fisher): | 3.3 % | 3.5 % |

### Example 2

**Composition of 1000 capsules:**

| | |
|---|---|
| Amlodipine besylate | 6.95 g |
| Bisoprolol fumarate | 5.0 g |
| Microcrystalline cellulose | 30.05 g |
| Sodium starch glycolate | 2.0 g |
| Magnesium stearate | 2.0 g |
| Colloidal silicon | 1.0 g |

### Process:

Both active ingredients are sieved using a screen of 250 µm mesh, then homogenized with microcrystalline cellulose, sodium starch glycolate and colloid silica in a drum mixer for ten minutes. Then the magnesium stearate is added to the powder mixture and the obtained mixture is homogenised for further two minutes.

The homogenizate is filled into capsules having 47 mg filling substance using a tablet pressing machine.

The capsules are packaged into a cold-formed blister of OPA/AL/PVC composite foil of 130 µm thickness and covered with an aluminium lidding foil of 20 µm thickness.

The contaminant examination results of the capsules prepared and packaged according to the Example 2 at the manufacturing date and after 3 months storage at 40 °C/ 75% relative humidity are as follows:

| Contaminant | At the production date | After 3 months (40 °C/75 % relative humidity) | After 6 months (40 °C/75 % relative humidity) |
|---|---|---|---|
| N-(2-{[4-(2-clorphenyl)-3-(ethoxycarbonyl)-5-(methoxycarbonyl)-6-methyl-1,4-dihydro-2-pyridinyl]-methoxy}-ethyl)-asparaginic acid | < 0.02 % | 0.11 % | 0.23 % |

### Reference example:

**Composition for 1000 tablets:**

| | |
|---|---|
| Amlodipine besylate | 13.9 g |
| Bisoprolol fumarate | 10.0 g |
| Potassium hydrogen phosphate x 2H₂O (Emcompress) | 189.0 g |
| Maize starch (STA RX-1500) | 40.1 g |
| Povidon K-25 | 14.0 g |
| Crosspovidon | 10.0 g |
| Magnesium stearate | 2.0 g |
| Colloid silica | 1.0 g |

### Process:

Both active ingredients are sieved using a screen of 250 µm mesh, then homogenized with Emcompres, maize starch, povidon, crospovidone and colloid silica in a drum mixer for ten minutes. Then the magnesium stearate is added to the powder mixture and the obtained mixture is homogenised for further two minutes.
The homogenizate is pressed into tablets having 280 mg weight with a tablet pressing machine.

The tablets are packaged into a thermoformed blister of a PVC foil of 250 µm thickness and a coating layer of 60g/m² of PVdC and the blister is covered with an aluminium lidding foil of 20 µm thickness.

The contaminant examination results of the tablets prepared and packaged according to the Reference Example at the manufacturing date and after 3 months storage at 40 °C/ 75% relative humidity are as follows:

| Contaminant | At the production date | After 3 months (40 °C/75 % relative humidity) |
|---|---|---|
| N-(2-{[4-(2-clorphenyl)-3-(ethoxycarbonyl)-5-(methoxycarbonyl)-6-methyl-1,4-dihydro-2-pyridinyl]-methoxy}-ethyl)-asparaginic acid | < 0.02 % | 0.47 % |

Contaminations of the tablets according to the Reference example are considerably higher after 3 months storage, resulting partly from the chemical properties of the used excipients (humidity and chemical incompatibility), partly from the insufficient damp-proof properties of the packaging system.

## Claims

1. Stable solid pharmaceutical composition containing a homogenized powder mixture of amlodipine base or a pharmaceutically acceptable salt thereof and bisoprolol fumarate and pharmaceutically accepted excipients packaged in a damp-proof package and further comprising less than 0.5 % of the compound of the formula 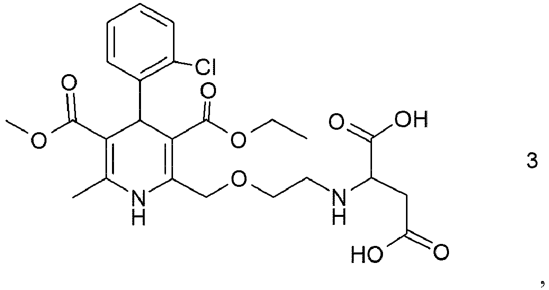 based on the weight of the active ingredients, wherein the composition further comprises an organic or inorganic filler, disintegrant, lubricant and optionally a binding agent.

2. Pharmaceutical composition according to Claim 1, **characterized in that** the composition contains amlodipine besylate.

3. Pharmaceutical composition according to Claim 1, **characterized in that** the composition contains less than 0.3 % of the compound of the formula (3).

4. Pharmaceutical composition according to Claim 1, **characterized in that** the composition contains less than 0.2 % of the compound of the formula (3).

5. Pharmaceutical composition according to Claim 1, **characterized in that** the composition is packaged into a cold-formed blister (so-called cold blister /CFF/) of OPA/AL/PVC composite foil covered with an aluminium lidding foil.

6. Pharmaceutical composition according to Claim 1, **characterized in that** the composition is packaged into a blister of thermoformed damp-proof composite foil and covered with an aluminium lidding foil.

7. Pharmaceutical composition according to Claim 1, **characterized in that** the composition is packaged into a glass or polypropylene vessel equipped with an airtight container cap of polyethylene or polypropylene.

8. Pharmaceutical composition according to any of Claims 1-7, which is a tablet or a capsule.

9. Pharmaceutical composition according to Claim 8, which is a tablet packaged in a damp-proof package, **characterised in that** it comprises 2%-20%, preferably 2%-10%, more preferably, 1-6% of amlodipine besylate, 2%-20%, preferably 2%-10%, more preferably 1%-6% of bisoprolol fumarate, further comprises 60%-90%, preferably 70%-90%, more preferably 80%-90% of filler, 1%-10%, preferably 4%-6% of disintegrant, 0.5%-3%, preferably 1%-2% of lubricant, 0.3%-2%, preferably 0.1%-5% of binding agent based on the weight of the tablet.

10. Pharmaceutical composition according to Claim 1, which is a tablet packaged into a cold-formed blister (so-called cold blister /CFF/) of OPA/AL/PVC composite foil covered with an aluminium lidding foil, or into a blister of thermoformable, damp-proof composite foil and covered with an aluminium lidding foil, or into a glass or polypropylene vessel equipped with an airtight container cap of polyethylene or polypropylene, comprising 1%-6% of amlodipine besylate, 1%-6% of bisoprolol fumarate, 80%-90% of microcrystalline cellulose, 4%-6% of sodium starch glycolate, 1%-2% of magnesium stearate, 0.5%-1% of colloid silica based on the weight of the tablet.

11. Pharmaceutical composition according to Claim 1, which is a capsule packaged into a cold-formed blister (so-called cold blister /CFF/) of OPA/AL/PVC composite foil covered with an aluminium lidding foil, or into a blister of thermoformable damp-proof composite foil and covered with an aluminium lidding foil, or into a glass or polypropylene vessel equipped with an airtight container cap of polyethylene or polypropylene, comprising 10%-15% of amlodipine besylate, 10%-15% of bisoprolol fumarate, further 55%-65% of microcrystalline cellulose, 4%-6% of sodium starch glycolate, 1%-2% of magnesium stearate, 0.5%-1% of colloid silica based on the weight of the filling substance of the capsule.

12. Process for the preparation of a stable solid pharmaceutical composition containing amlodipine base or a pharmaceutically acceptable salt thereof and bisoprolol fumarate, **characterized in that** the amlodipine base or a pharmaceutically acceptable salt thereof and the bisoprolol fumarate, a disintegrant, a lubricant and if necessary further excipients used in the pharmaceutical industry are homogenised, then an anti-adhesive is added, the homogenisation is continued, then
a.) the homogenizate is pressed into tablets using a direct compressing process or,
b.) filled into hard gelatine capsules in a known manner,
then the obtained tablets or capsules are packaged into damp-proof protecting packages in a known manner.

13. Process for the preparation of tablets according to Claim 12, **characterised in that** 2%-20%, preferably 2%-10%, more preferably 1-6% of amlodipine base or a pharmaceutically accepted acid addition salt thereof, preferably amlodipine besylate, 2%-20%, preferably 2%-10%, more preferably 1%-6% of bisoprolol fumarate, further 60%-90%, preferably 70%-90%, more preferably 80%-90% of filler, 1%-10%, preferably 4%-6% of disintegrant, 0.5%-3%, preferably 1%-2% of lubricant, 0.3%-2%, preferably 1%-10%, preferably 0.5%-1% of binding agent are used based on the weight of the tablets.

14. Process for the preparation of capsules according to Claim 12, **characterised in that** 5%-80%, preferably 5%-18%, more preferably, 10-15% of amlodipine base or a pharmaceutically accepted acid addition salt thereof, preferably amlodipine besylate, 5%-80%, preferably 5%-15%, more preferably 10%-15% of bisoprolol fumarate, further 1%-10%, preferably 4%-6% of disintegrant, 0.5%-3%, preferably 1%-2% of lubricant, 0.3%-2%, preferably 0.5%-1% of anti-adhesive agent, and if necessary 10%-75%, preferably 45%-75%, more preferably 55%-65% of filler are used based on the weight of the filling substance of the capsule.

## Patentansprüche

1. Stabile feste pharmazeutische Zusammensetzung, umfassend eine homogenisierte Pulvermischung aus Amlodipinbase oder einem pharmazeutisch akzeptablen Salz davon und Bisoprololfumarat und pharmazeutisch akzeptablen Hilfsstoffen, verpackt in einer feuchtigkeitsbeständigen Verpackung und ferner umfassend weniger als 0,5 % der Verbindung der Formel 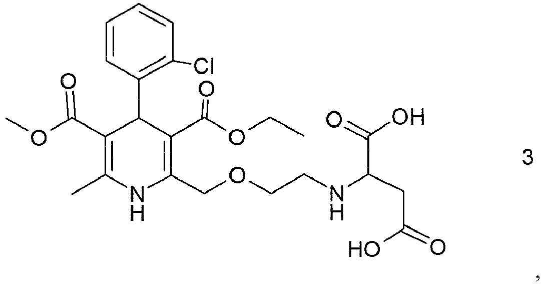 bezogen auf das Gewicht der Wirkstoffe, wobei die Zusammensetzung ferner einen organischen oder anorganischen Füllstoff, Desintegrant, Gleitmittel und optional ein Bindemittel umfasst.

2. Pharmazeutische Zusammensetzung nach Anspruch 1, **dadurch gekennzeichnet, dass** die Zusammensetzung Amlodipinbesylat umfasst.

3. Pharmazeutische Zusammensetzung nach Anspruch 1, **dadurch gekennzeichnet, dass** die Zusammensetzung weniger als 0,3 % der Verbindung der Formel (3) umfasst.

4. Pharmazeutische Zusammensetzung nach Anspruch 1, **dadurch gekennzeichnet, dass** die Zusammensetzung weniger als 0,2 % der Verbindung der Formel (3) umfasst.

5. Pharmazeutische Zusammensetzung nach Anspruch 1, **dadurch gekennzeichnet, dass** die Zusammensetzung in einen kaltgeformten Blister (sogenannter Cold Blister /CFF/) aus OPA/AL/PVC-Verbundfolie verpackt ist, der mit einer Aluminium-Deckfolie abgedeckt ist.

6. Pharmazeutische Zusammensetzung nach Anspruch 1, **dadurch gekennzeichnet, dass** die Zusammensetzung in einem Blister aus thermogeformter, feuchtigkeitsbeständiger Verbundfolie verpackt und mit einer Aluminium-Deckfolie abgedeckt ist.

7. Pharmazeutische Zusammensetzung nach Anspruch 1, **dadurch gekennzeichnet, dass** die Zusammensetzung in ein Glas- oder Polypropylengefäß verpackt ist, das mit einem luftdichten Behälterdeckel aus Polyethylen oder Polypropylen versehen ist.

8. Pharmazeutische Zusammensetzung nach einem der Ansprüche 1-7, die eine Tablette oder eine Kapsel ist.

9. Pharmazeutische Zusammensetzung nach Anspruch 8, die eine Tablette ist, die in einer feuchtigkeitsbeständigen Verpackung verpackt ist, **dadurch gekennzeichnet, dass** sie 2%-20%, vorzugsweise 2%-10%, bevorzugter 1-6% Amlodipinbesylat, 2%-20%, vorzugsweise 2%-10%, bevorzugter 1%-6% Bisoprololfumarat umfasst, ferner 60%-90%, vorzugsweise 70%-90%, bevorzugter 80%-90% Füllstoff, 1%-10%, vorzugsweise 4%-6% Desintegrant, 0,5%-3%, vorzugsweise 1%-2% Gleitmittel, 0,3%-2%, vorzugsweise 0,1%-5% Bindemittel bezogen auf das Gewicht der Tablette umfasst.

10. Pharmazeutische Zusammensetzung nach Anspruch 1, die eine Tablette ist, die in einen kaltgeformten Blister (sogenannter Cold Blister /CFF/) aus OPA/AL/PVC-Verbundfolie, der mit einer Aluminium-Deckfolie abgedeckt ist, oder in einen Blister aus thermoformbarer, feuchtigkeitsbeständiger Verbundfolie und mit einer Aluminium-Deckfolie abgedeckt ist, oder in ein Glas- oder Polypropylengefäß, das mit einem luftdichten Behälterdeckel aus Polyethylen oder Polypropylen versehen ist, verpackt ist, umfassend 1%-6% Amlodipinbesylat, 1%-6% Bisoprololfumarat, 80%-90% mikrokristalline Cellulose, 4%-6% Natriumstärkeglykolat, 1%-2% Magnesiumstearat, 0,5%-1% kolloidales Siliciumdioxid bezogen auf das Gewicht der Tablette.

11. Pharmazeutische Zusammensetzung nach Anspruch 1, welche eine Kapsel ist, die in einen kaltgeformten Blister (sogenannter Cold Blister /CFF/) aus OPA/AL/PVC-Verbundfolie, der mit einer Aluminium-Deckfolie abgedeckt ist, oder in einen Blister aus thermoformbarer, feuchtigkeitsbeständiger Verbundfolie und mit einer Aluminium-Deckfolie abgedeckt ist, oder in ein Glas- oder Polypropylengefäß, das mit einem luftdichten Behälterdeckel aus Polyethylen oder Polypropylen versehen ist, verpackt ist, umfassend 10%-15% Amlodipinbesylat, 10%-15% Bisoprololfumarat, ferner 55%-65% mikrokristalline Cellulose, 4%-6% Natriumstärkeglykolat, 1%-2% Magnesiumstearat, 0,5%-1% kolloidales Siliciumdioxid bezogen auf das Gewicht der Füllsubstanz der Kapsel.

12. Verfahren zur Herstellung einer stabilen festen verpackten Darreichungsform, umfassend Amlodipinbase oder ein pharmazeutisch akzeptables Salz davon und Bisoprololfumarat, **dadurch gekennzeichnet, dass** die Amlodipinbase oder ein pharmazeutisch akzeptables Salz davon und das Bisoprololfumarat, ein Desintegrant, ein Gleitmittel und, wenn nötig, weitere in der pharmazeutischen Industrie verwendete Hilfsstoffe homogenisiert werden, dann ein Antihaftmittel zugegeben wird, die Homogenisierung fortgesetzt wird, dann
a.) das Homogenisat in Tabletten mittels eines direkten Komprimierungsverfahrens gepresst wird oder,
b.) in Hartgelatinekapseln in bekannter Weise abgefüllt wird,
wobei dann die erhaltenen Tabletten oder Kapseln in einer bekannten Weise in feuchtigkeitsbeständige Schutzverpackungen verpackt werden.

13. Verfahren zur Herstellung von Tabletten nach Anspruch 12, **dadurch gekennzeichnet, dass** 2%-20%, vorzugsweise 2%-10%, bevorzugter 1-6% Amlodipinbase oder ein pharmazeutisch akzeptables Säureadditionssalz davon, vorzugsweise Amlodipinbesylat, 2%-20%, vorzugsweise 2%-10%, bevorzugter 1%-6% Bisoprololfumarat, ferner 60%-90%, vorzugsweise 70%-90%, bevorzugter 80%-90% Füllstoff, 1%-10%, bevorzugter 4%-6% Desintegrant, 0,5%-3%, vorzugsweise 1%-2% Gleitmittel, 0,3%-2%, vorzugsweise 1%-10%, vorzugsweise 0,5%-1% Bindemittel verwendet werden bezogen auf das Gewicht der Tabletten.

14. Verfahren zur Herstellung von Kapseln nach Anspruch 12, **dadurch gekennzeichnet, dass** 5%-80%, vorzugsweise 5%-18%, bevorzugter 10-15% Amlodipinbase oder ein pharmazeutisch akzeptables Säureadditionssalz davon, vorzugsweise Amlodipinbesylat, 5%-80%, vorzugsweise 5%-15%, bevorzugter 10%-15% Bisoprololfumarat, ferner 1%-10%, vorzugsweise 4%-6% Desintegrant, 0,5%-3%, vorzugsweise 1%-2% Gleitmittel, 0,3%-2%, vorzugsweise 0,5%-1% Antihaftmittel und, wenn nötig, 10%-75%, vorzugsweise 45%-75%, bevorzugter 55%-65% Füllstoff verwendet werden bezogen auf das Gewicht der Füllsubstanz der Kapsel.

## Revendications

1. Composition pharmaceutique solide stable contenant un mélange en poudre homogénéisé de base amlodipine ou d'un de ses sels pharmaceutiquement acceptables et de fumarate de bisoprolol et d'excipients pharmaceutiquement acceptables conditionnés dans un emballage résistant à l'humidité et comprenant en outre moins de 0,5% du composé de formule 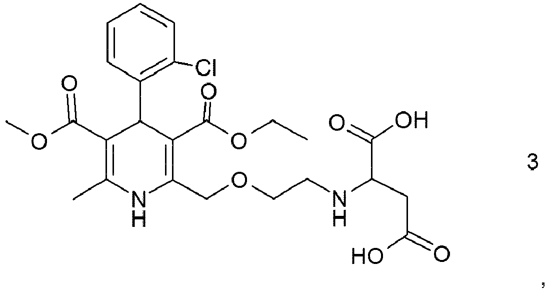 sur la base du poids des ingrédients actifs, la composition comprenant en outre une charge organique ou inorganique, un désintégrant, un lubrifiant et éventuellement un agent liant.

2. Composition pharmaceutique selon la revendication 1, **caractérisée en ce que** la composition contient du bésylate d'amlodipine.

3. Composition pharmaceutique selon la revendication 1, **caractérisée en ce que** la composition contient moins de 0,3% du composé de formule (3).

4. Composition pharmaceutique selon la revendication 1, **caractérisée en ce que** la composition contient moins de 0,2% du composé de formule (3).

5. Composition pharmaceutique selon la revendication 1, **caractérisée en ce que** la composition est conditionnée dans un blister formé à froid (appelé «blister à froid» /CFF/) en feuille composite OPA/AL/PVC recouverte d'une feuille de couverture en aluminium.

6. Composition pharmaceutique selon la revendication 1, **caractérisée en ce que** la composition est conditionnée dans un blister en feuille composite thermoformée résistant à l'humidité et recouvert d'une feuille de couverture en aluminium.

7. Composition pharmaceutique selon la revendication 1, **caractérisée en ce que** la composition est conditionnée dans un récipient en verre ou en polypropylène équipé d'un opercule de récipient étanche à l'air en polyéthylène ou en polypropylène.

8. Composition pharmaceutique selon l'une quelconque des revendications 1 à 7, qui est un comprimé ou une capsule.

9. Composition pharmaceutique selon la revendication 8, qui est un comprimé conditionné dans un emballage résistant à l'humidité, **caractérisée en ce qu'**elle comprend 2%-20%, de préférence 2%-10%, plus préférablement 1-6% de bésylate d'amlodipine, 2%-20%, de préférence 2%-10%, plus préférablement 1%-6% de fumarate de bisoprolol, comprend en outre 60%-90%, de préférence 70%-90%, plus préférablement 80%-90% de charge, 1%-10%, de préférence 4%-6% de désintégrant, 0,5%-3%, de préférence 1%-2% de lubrifiant, 0,3%-2%, de préférence 0,1%-5% de liant sur la base du poids du comprimé.

10. Composition pharmaceutique selon la revendication 1, qui est un comprimé conditionné dans un blister formée à froid (appelée «blister à froid» /CFF/) en feuille composite OPA/AL/PVC recouvert d'une feuille de couverture en aluminium ou en un blister de feuille composite thermoformable résistant à l'humidité et recouvert d'une feuille de couverture en aluminium, ou dans un récipient en verre ou en polypropylène équipé d'un opercule étanche à l'air en polyéthylène ou en polypropylène, contenant 1%-6% de bésylate d'amlodipine, 1%-6% de bisoprolol fumarate, 80%-90% de cellulose microcristalline, 4%-6% de glycolate d'amidon sodique, 1%-2% de stéarate de magnésium, 0,5%-1% de silice colloïdale sur la base du poids du comprimé.

11. Composition pharmaceutique selon la revendication 1, qui est une capsule conditionnée dans un blister formée à froid (appelée «blister à froid» /CFF/) en feuille composite OPA/AL/PVC recouvert d'une feuille de couverture en aluminium ou dans une blister de feuille composite thermoformable résistant à l'humidité et recouvert d'une feuille de couverture en aluminium ou dans un récipient en verre ou en polypropylène équipé d'un opercule étanche à l'air en polyéthylène ou en polypropylène, contenant 10%-15% de bésylate d'amlodipine, 10%-15% de bisoprolol fumarate, 55%-65% de cellulose microcristalline, 4%-6% de glycolate d'amidon sodique, 1%-2% de stéarate de magnésium, 0,5%-1% de silice colloïdale sur la base du poids de la substance de remplissage de la capsule .

12. Procédé de préparation stable composition pharmaceutique solide et conditionnée contenant de l'amlodipine base ou d'un de ses sels pharmaceutiquement acceptables et du fumarate de bisoprolol, **caractérisée en ce que** la base amlodipine ou un de ses sels pharmaceutiquement acceptables et le fumarate de bisoprolol, un désintégrant, un lubrifiant et, si nécessaire, d'autres excipients utilisés dans l'industrie pharmaceutique sont homogénéisés, puis un anti-adhésif est ajouté, l'homogénéisation est poursuivie, puis
a.) l'homogénéisate est pressé en comprimés en utilisant un procédé de compression directe ou,
b.) remplie de manière connue en capsules de gélatine dure,
ensuite, les comprimés ou capsules obtenus sont emballés dans des emballages protecteurs résistant à l'humidité de manière connue.

13. Procédé de préparation de comprimés selon la revendication 12, **caractérisé en ce que** 2%-20%, de préférence 2%-10%, plus préférablement 1-6% d'amlodipine base ou d'un de ses sels d'addition d'acide pharmaceutiquement acceptables, de préférence d'amlodipine bésylate, 2%-20%, de préférence 2%-10%, plus préférablement 1%-6% de fumarate de bisoprolol, encore 60%-90%, de préférence 70%-90%, plus préférablement 80%-90% de charge, 1%-10%, de préférence 4%-6% de désintégrant, 0,5%-3%, de préférence 1%-2% de lubrifiant, 0,3%-2%, de préférence 1%-10%, de préférence 0,5%-1 % de liant sont utilisés sur la base du poids des comprimés.

14. Procédé de préparation de capsules selon la revendication 12, **caractérisé en ce que** 5%-80%, de préférence 5%-18%, plus préférablement 10-15% de l'amlodipine base ou de l'un de ses sels d'addition d'acide pharmaceutiquement acceptés, de préférence l'amlodipine bésylate, 5%-80%, de préférence 5%-15%, plus préférablement 10%-15% de fumarate de bisoprolol, encore 1%-10%, de préférence 4%-6% de désintégrant, 0,5%-3%, de préférence 1%-2% de lubrifiant, 0,3%-2%, de préférence 0,5%-1% d'agent anti-adhésif, et si nécessaire 10%-75%, de préférence 45%-75%, plus préférablement 55%-65% de charge sont utilisés sur la base du poids de la substance de remplissage de la capsule.
